(19) Europäisches Patentamt
European Patent Office
Office européen des brevets

(11) Numéro de publication : **0 527 067 A1**

# (12) DEMANDE DE BREVET EUROPEEN

(21) Numéro de dépôt : **92401834.4**

(22) Date de dépôt : **29.06.92**

(51) Int. Cl.$^5$ : **C07C 69/24,** C07C 69/007, C07C 67/08, C09K 15/06

(30) Priorité : **25.07.91 FR 9109694**

(43) Date de publication de la demande : **10.02.93 Bulletin 93/06**

(84) Etats contractants désignés : **AT BE CH DE DK ES FR GB GR IT LI LU NL PT SE**

(71) Demandeur : **RHONE-POULENC CHIMIE**
**25, quai Paul Doumer**
**F-92408 Courbevoie Cédex (FR)**

(72) Inventeur : **Chicart, Philippe**
**33 rue du Phare Sainte-Marine**
**F-29120 Combrit (FR)**
Inventeur : **Gay, Michel**
**10 rue Henri Rolland**
**F-69100 Villeurbanne (FR)**

(74) Mandataire : **CABINET PLASSERAUD**
**84; rue d'Amsterdam**
**F-75009 Paris (FR)**

## (54) Nouvelles benzophénones à fonction ester et leur utilisation dans les polymères.

(57) La présente invention concerne de nouvelles hydroxybenzophénones à fonction ester, ainsi que l'utilisation de ces composés comme absorbeurs UV dans les polymères organiques.

Ce sont plus précisément des carboxylates du di(benzoyl-4 hydroxy-3 phénoxy)-1,3 propan-2-ol qui ont de préférence une masse moléculaire d'au moins 500.

Ces benzophénones peuvent être utilisées comme anti-UV dans les polymères organiques, soit seules, soit conjointement avec des benzophénones connues de masse moléculaire plus faible, telles que les hydroxy-2 alcoxy-4 benzophénones.

EP 0 527 067 A1

La présente invention concerne de nouvelles hydroxybenzophénones à fonction ester, ainsi que l'utilisation de ces composés comme absorbeurs UV dans les polymères organiques.

Les hydroxy-2 benzophénones sont des absorbeurs UV très largement utilisés pour la protection des polymères contre les effets néfastes de la lumière.

Ce sont des composés efficaces. Cependant ils sont parfois trop rapidement éliminés lors de la mise en forme ou de certaines utilisations des polymères, en raison de leur volatilité excessive et la protection de longue durée des polymères exposés à la lumière pose certains problèmes.

Il a été proposé dans le brevet JP-A-89.96259 de greffer des motifs hydroxy-2 benzophénone sur des chaînes polysiloxaniques. La synthèse de ces composés est cependant relativement complexe et le degré souhaité de greffage des motifs hydroxy-2 benzophénone n'est pas toujours facile à atteindre précisément.

La présente invention a trait à des composés simples comportant au moins 2 motifs hydroxy-2 benzophénone et ayant une masse moléculaire d'au moins 500.

Ce sont plus précisément des benzophénones de formule générale (I)

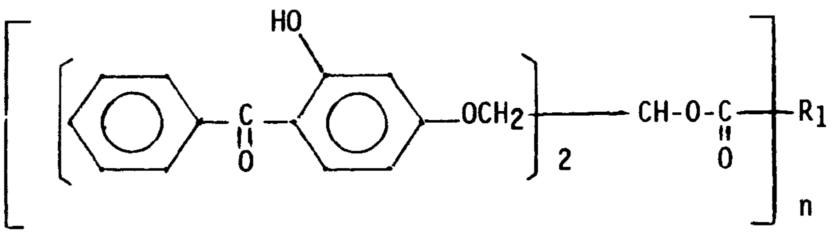

(I)

dans laquelle :

- n représente 1 ou 2
- $R_1$ , lorsque n est égal à 1,de 6 à 40 , avantageusement de 12 à 40 de préference de 14 à 30 atomes de carbone et represente:
  . un radical alkyle
  . un radical phényle,
  . un radical phényle comportant 1 ou 2 substituants alkyles, linéaires ou ramifiés, ayant de 1 à 6 atomes de carbone, et/ou un radical hydroxyle,

et si n=2

- $R_1$ Présente de 6 à 40 avantageusement de 12 à 40 de préference de 14 à 40 atomes de carbone et représente :

un radical alkyle;
  . un radical orthophénylène, métaphénylène ou paraphénylène,
  . un radical orthophénylène, métaphénylène ou paraphénylène, comportant 1 ou 2 substituants alkyles, linéaires ou ramifiés, ayant de 1 à 6 atomes de carbone,
  . un radical cyclohexane orthodialkylène, dont les radicaux alkylènes, identiques aux différents linéaires ou ramifiés, comportent de 1 à 12 atomes de carbone,
  . un radical orthocyclohexylène, métacyclohexylène ou paracyclohexylène,
  . un radical cyclohexane-dialkylène ou cyclohexylène tel que défini précédemment comportant 1 ou 2 substituants alkyles, linéaires ou ramifiés, ayant 1 à 6 atomes de carbone.

Les nouvelles benzophénones à fonction ester de l'invention sont plus particulièrement les composés de formule générale (I) dans laquelle :

- n représente 1 ou 2,
- $R_1$ représente lorsque n = 1, un radical alkyle, linéaire ou ramifié, ayant de 5 à 30 atomes de carbone, tel que par exemple pentyles, hexyles, heptyles, octyles, nonyles, décyles, undécyles, dodécylés, tridécyles, tétradécyles, pentadécyles, heptadécyles, nonadécyles,
- $R_1$ représente, lorsque n = 2 :
  . un radical alkylène, linéaire ou ramifié, ayant 1 à 24 atomes de carbone, tel que par exemple méthylène, éthylène, triméthylène, tétraméthylène, pentaméthylène, hexaméthylène, heptaméthylène, octaméthylène, nonaméthylène, décaméthylène, dodécaméthylène, tétradécaméthylène, hexadécaméthylène, octadécaméthylène,
  . un radical cyclohexane-orthodioctaméthylène comportant 2 substituants alkyles,linéaires ou ramifiés ayant 6 atomes de carbone.

A titre d'exemples non limitatifs de benzophénones à fonction ester de formule (I), ont peut citer :

- le décanoate de di(benzoyl-4 hydroxy-3 phénoxy)-1,3 propyle-2,
- le dodécanoate (laurate) de di(benzoyl-4 hydroxy-3 phénoxy)-1,3 propyle-2,
- l'hexadécanoate de di(benzoyl-4 hydroxy-3 phénoxy)-1,3 propyle-2,
- l'octadécanoate (stéarate) de di(benzoyl-4 hydroxy-3 phénoxy)-1,3 propyle-2,
- le docosanoate (béhénate) de di(benzoyl-4 hydroxy-3 phénoxy)-1,3 propyle-2,
- le (dihexyl-4,5 cyclohexane)-di(octaméthylène-carboxylate)-1,2 de bis[di(benzoyl-4 hydroxy-3 phénoxy)-1,3 propyle-2].

Les benzophénones à fonction ester de formule (I) peuvent être préparées en faisant réagir le di(benzoyl-4 hydroxy-3 phénoxy)-1,3 propan-2 ol de formule (II) :

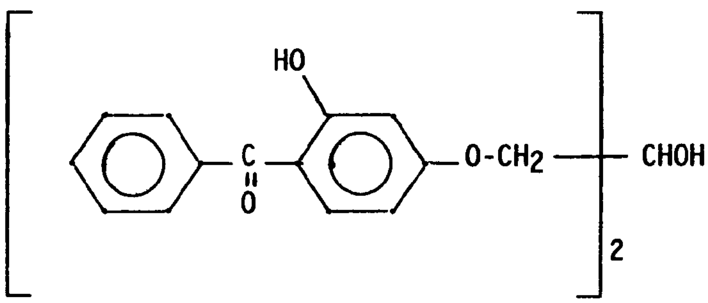

(II)

avec un acide de formule générale (III) :

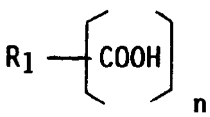

(III)

dans laquelle :

- n représente 1 ou 2
- $R_1$ a des significations indiquées précédemment pour les composés de formule générale (I),

en présence d'un catalyseur acide.

Le di(benzoyl-4 hydroxy-3 phénoxy)-1,3 propan-2 ol de formule (II) est un composé connu dont la préparation est décrite par exemple dans le brevet SU-A-277764.

Le catalyseur acide utilisé pour la réaction d'estérification de l'alcool de formule (II) par l'acide $R_1$-$(COOH)_n$ peut être tout catalyseur acide usuel de ce type de réaction.

Le plus fréquemment utilisé est l'acide paratoluènesulfonique.

Généralement la réaction est effectuée dans un solvant permettant d'éliminer par distillation azéotropique l'eau formée. Ainsi on peut utiliser par exemple le toluène, le benzène ou les xylènes.

La réaction est conduite à une température de 50°C à 160°C et de préférence de 60°C à 130°C.

En pratique, il est commode d'opérer à une température correspondant à la température de reflux du solvant utilisé.

La réaction dure généralement plusieurs heures.

Les benzophénones de formule (I) peuvent être utilisées notamment comme absorbeurs UV dans les polymères organiques.

Ainsi elles peuvent être utilisées comme absorbeurs UV dans les polyoléfines, les polystyrènes, les polyalcadiènes, les polyuréthannes, les polyéthers-sulfones, les polyéthers-cétones, les polymères acryliques, les polymères halogénés, leurs copolymères et leurs mélanges.

Un autre objet de l'invention consiste donc dans des compositions de polymère organique stabilisé par une quantité efficace d'au moins une benzophénone de formule (I).

Il est préféré dans cette application d'utiliser des benzophénones de formule (I) ayant une masse moléculaire égale ou supérieure à 600.

Une variante intéressante de l'invention consiste dans l'utilisation conjointe d'au moins une benzophénone de formule (I) et d'une benzophénone connue de masse moléculaire plus faible.

Cette variante de la présente invention concerne donc des compositions de polymère organique stabilisé par une quantité efficace d'au moins une benzophénone de formule (I) et d'au moins une benzophénone de formule générale (IV) :

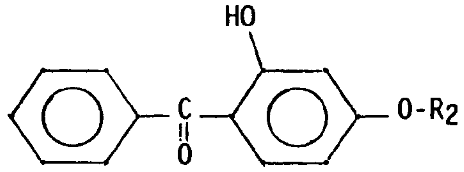

(IV)

dans laquelle $R_2$ représente un radical alkyle, linéaire ou ramifié, ayant de 1 à 30 atomes de carbone.

Habituellement les compositions de l'invention contiennent :

- soit de 0,005 % à 5 % en poids de benzophénone de formule (I) par rapport au poids du polymère à stabiliser et de préférence de 0,01 % à 2 % en poids par poids,
- soit de 0,005 % à 5 % en poids d'un mélange de benzophénone de formule (I) et de benzophénone de formule (IV) par rapport au poids du polymère à stabiliser et de préférence de 0,01 % à 2 % en poids par poids.

Il est clair que les compositions stabilisées de polymère organique peuvent contenir un mélange de plusieurs composés de formule (I).

L'addition des composés de formule (I) ou d'un mélange de composés de formule (I) et de composés de formule (IV) peut être effectuée pendant ou après la préparation des polymères.

Ces compositions de polymères organiques contenant les benzophénones de formule (I) ou des mélanges de benzophénones de formule (I) et de benzophénones de formule (IV) peuvent contenir en outre les additifs et stabilisants habituellement utilisés tels que

- les antioxydants, en particulier les esters de l'acide 3-(3,5-di-t-butyl-4-hydroxy-phénmyl)propionique et plus particulièrement le tétrakis[3-(3,5-di-tertio-butyl-4-hydroxyphényl)-propionyloxyméthyl]méthane et le 3-(3,5-di-t-butyl-4-hydroxyphényl)-propionate de stéaryle ;
- les Hals (stabilisant lumière : amine encombrée) dérivés de la 2,2,6,6-tétraméthylpipéridine et plus particulièrement le bis(2,2,6,6-tétraméthyl-4-pipéridyl)sébacate ;
- les phosphites et phosphonites et plus particulièrement le phosphite de tris(ditertiobutyl-2,4-phényle) ;
- les désactiveurs de métaux ;
- les composés destructeurs de peroxydes, en particulier les thioéthers ;
- les agents de nucléation :
- les charges et agents de renforcement ;
- les plastifiants ;
- les lubrifiants ;
- les émulsionnants ;
- les pigments ;
- les azurants optiques ;
- les ignifugeants ;
- les antistatiques ;
- les porogènes.

Les compositions de polymères stabilisées peuvent être mises en oeuvre sous les formes les plus variées, par exemple sous forme d'objets moulés, de feuilles, de fibres, de matériaux cellulaires (masses), de profilés ou de produits de revêtement, ou comme feuillogènes (liants) pour pentures, vernis, colles ou ciments.

Les exemples qui suivent illustrent l'invention.

## EXEMPLE 1

Préparation du produit (Ia) : ester stéarique du di-(benzoyl-4 hydroxy-3 phénoxy)-1,3 propan-2-ol.

Composé de formule générale (I) dans laquelle

$$R_1 = -(CH_2)_{16}-CH_3$$

Dans un ballon tricol de 500 cm³, équipée d'une agitation centrale, d'un thermomètre et d'un "dean-stark" surmonté d'un réfrigérant sous ciel d'azote, on charge :

- 45,0 g (0,093 mol) d'alcool de formule (II),
- 24,0 g (0,084 mol) d'acide stéarique,
- 1,0 g d'acide paratoluènesulfonique,
- 350 cm³ de toluène.

Le mélange réactionnel est ensuite porté au reflux du toluène pendant 24 heures. Après retour à la température ambiante, le mélange réactionnel est lavé trois fois avec 150 cm³ d'eau. La phase organique, non

homogène, est filtrée (le solide récupéré est un mélange d'alcool et d'acide de départ n'ayant pas réagi). La phase organique est ensuite séchée sur sulfate de sodium, et le solvant est évaporé sous pression réduite. Les 50,5 g de produit brut obtenus sont chromatographiés sur une colonne de gel de silice en élution hexane/acétate d'éthyle 2/1 pour éliminer les restes d'alcool et d'acide contenus dans le produit.

On obtient finalement 29,5 g d'un solide jaune pâle (soit un rendement en produit isolé pur de 47 % par rapport à l'acide de départ) dont la structure est confirmée par spectrométrie de masse et par résonance magnétique du proton ($RMN^1H$) : pureté supérieure à 95 %.

Caractéristiques physiques

- Point de fusion : 82°C
- UV (solvant $CHCl_3$) :
  - $^{\varepsilon}327nm = 19290\ l.mol^{-1}.cm^{-1}$
  - $^{\varepsilon}287nm = 30790\ l.mol^{-1}.cm^{-1}$

EXEMPLE 2

Préparation du produit (Ib) : docosanoate (béhénate) du di(benzoyl-4-hydroxy-3 phénoxy)-1,3 propan-2-ol.

Composés de formule générale (I) dans laquelle

$$R_1 = -(CH_2)_{20}-CH_3$$

Dans un ballon tricol de 500 cm$^3$, équipée d'une agitation centrale, d'un thermomètre et d'un "dean-stark" surmonté d'un réfrigérant sous ciel d'azote, on charge :

- 26,6 g (0,055 mol) d'alcool de formule (II),
- 17,0 g (0,050 mol) d'acide béhénique technique (acide docosanoïque majoritaire)
- 1,0 g d'acide paratoluènesulfonique,
- 250 cm$^3$ de toluène.

Le mélange réactionnel est ensuite porté au reflux du toluène pendant 24 heures. Après retour à la température ambiante, le mélange réactionnel est lavé trois fois avec 150 cm$^3$ d'eau. La phase organique, non homogène, est filtrée (le solide récupéré est un mélange d'alcool et d'acide de départ n'ayant pas réagi). La phase organique est ensuite séchée sur sulfate de sodium, et le solvant est évaporé sous pression réduite. Les 31,0 g de produit brut obtenus sont chromatographiés sur une colonne de gel de silice en élution hexane/acétate d'éthyle 2/1 pour éliminer les restes d'alcool et d'acide contenus dans le produit.

On obtient finalement 20,8 g d'un solide jaune pâle (soit un rendement en produit isolé pur de 52 % par rapport à l'acide de départ) dont la structure est confirmée par spectrométrie de masse et par résonance magnétique du proton ($RMN^1H$) : pureté supérieure à 95 %.

Caractéristiques physiques

- Point de fusion : 30°C
- UV (solvant $CHCl_3$) :
  - $^{\varepsilon}327nm = 18850\ l.mol^{-1}.cm^{-1}$
  - $^{\varepsilon}287nm = 30265\ l.mol^{-1}.cm^{-1}$

EXEMPLE 3

Préparation du produit (Ic) : estérification du di-(4-benzoyl hydroxy-3 phénoxy)-1,3 propan-2-ol par le diacide de formule (V) :

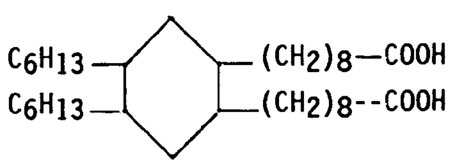

(V)

Dans un ballon tricol de 500 cm$^3$, équipée d'une agitation centrale, d'un thermomètre et d'un "dean-stark" surmonté d'un réfrigérant sous ciel d'azote, on charge :

- 25,0 g (0,052 mol) d'alcool de formule (II),
- 13,9 g (0,024 mol) du diacide (V)
- 1,0 g d'acide paratoluènesulfonique,
- 250 $cm^3$ de toluène.

Le mélange réactionnel est ensuite porté au reflux du toluène pendant 24 heures. Après retour à la température ambiante, le mélange réactionnel est lavé trois fois avec 150 $cm^3$ d'eau. La phase organique est ensuite séchée sur sulfate de sodium, et le solvant est évaporé sous pression réduite. Les 28,7 g de produit brut obtenus sont chromatographiés sur une colonne de gel de silice en élution hexane/acétate d'éthyle 2/1 pour éliminer les restes d'alcool et l'acide contenus dans le produit.

On obtient finalement 23,5 g d'un liquide visqueux jaune pâle (soit un rendement en produit isolé pur de 64 % par rapport à l'acide de départ) dont la structure est confirmée par résonance magnétique du proton ($RMN^1H$) : pureté de 90 %.

Caractéristiques physiques

- UV (solvant $CHCl_3$) :
  - $^{\varepsilon}327nm = 36310\ l.mol^{-1}.cm^{-1}$
  - $^{\varepsilon}287nm = 58320\ l.mol^{-1}.cm^{-1}$

EXEMPLE 4

Evaluations des produits (Ia) et (Ib) utilisés seuls ou en associations avec l'hydroxy-2-octyloxy-4 benzophénone (produit commercialisé sous la marque déposée RHODIALUX P) en vieillissement UV accéléré du polypropylène (PP).

Préparations des compositions

Dans un mélangeur lent, on prépare 100 g de chacun des mélanges dont la composition pondérale est indiquée ci-après :
- PP Appryl 3030 AP : 100 g
- Stéarate de calcium : 0,1 g
- Antioxydant Irganox 1076® : 0,05 g
- Absorbeur UV à évaluer : selon tableau 1

L'antioxydant commercialisé sous la marque IRGANOX 1076 est l'(hydroxy-4 ditertiobutyl-3,5 phényl)-3 propionate d'octadécyle.

Pressage

Les mélanges sont ensuite pressés deux fois à la presse Carver à 190°C : la première fois 5 min dont 3 sous 30 MPa (300 bars) et la seconde fois 5 min dont 3 sous 35 MPa (350 bars). Ceci permet d'obtenir des films de 50 µm. Dans les feuilles obtenues, sont découpés des échantillons de 12 x 30 mm.

Vieillissement

Ces échantillons soit placés dans une enceinte MPC/SEPAP 12/24 chauffée à 60°C et équipée de 4 lampes à vapeur de mercure "moyenne pression" du type MAZDA MA 400 W. La dégradation des films de polypropylène est suivie en infrarouge par l'apparition de la bande carbonyle à 1720 $cm^{-1}$. Les résultats sont donnés dans le tableau 1.

Les durées de vie en heures correspondent au temps mis pour atteindre une absorbance donnée. Dans notre cas, nous avons fixé log(Io/I) = 0,3.

| Compositions | Absorbeur U.V. (en g pour 100 g de PP) | Durée de vie |
|---|---|---|
| 4.1 | Rhodialux P 0,35 | 375 |
| 4.2 | Produit Ia 0,35 | 238 |
| 4.3 | Produit Ib 0,35 | 257 |
| 4.4 | Rhodialux P 0,18<br>Produit Ia 0,17 | 336 |
| 4.5 | Rhodialux P 0,18<br>Produit Ib 0,17 | 386 |
| 4.6 | Rhodialux P 0,25<br>Produit Ia 0,10 | 330 |
| 4.7 | Rhodialux P 0,25<br>Produit Ib 0,10 | 354 |
| 4.8 | Rhodialux P 0,30<br>Produit Ia 0,05 | 400 |
| 4.9 | Rhodialux P 0,30<br>Produit Ib 0,05 | 408 |

TABLEAU 1

EXEMPLE 5

Evaluations des produits (Ia) et (Ic) utilisés en associations avec le Rhodialux P et le Tinuvin 770 (décanoate de bis(2,2,6,6-tétraméthyl-4-pipéridinyle) en vieillissement UV accéléré du polypropylène.

Préparation des compositions

Dans un mélangeur lent, on prépare 100 g de chacun des mélanges dont la composition pondérale est indiquée ci-après :

- PP Appryl 3030 AP : 100 g
- Stéarate de calcium : 0,1 g
- Antioxydant Irganox 1076® : 0,05 g
- Hals Tinuvin 770® : 0,15 g
- Produits (Ia), (Ib) et Rhodialux P : selon tableau 2.

Le pressage des films et le vieillissement des échantillons sont effectués de la même manière que dans l'exemple 4. Les résultats sont donnés dans le tableau 2.

Les durées de vie en heures correspondent au temps mis pour atteindre une absorbance donnée. Dans notre cas, nous avons fixé log(Io/I) = 0,3.

| Compositions | Absorbeur UV (en g pour 100 g de PP) | Durée de vie |
|---|---|---|
| 5.1 | Témoin sans absorbeur UV | 241 |
| 5.2 | Rhodialux P 0,35 | 366 |
| 5.3 | Produit Ia 0,35 | 283 |
| 5.4 | Produit Ic 0,35 | 331 |
| 5.5 | Rhodialux P 0,30<br>Produit Ia 0,05 | 455 |
| 5.6 | Rhodialux P 0,30<br>Produit Ic 0,05 | 402 |
| 5.7 | Rhodialux P 0,25<br>Produit Ia 0,10 | 395 |
| 5.8 | Rhodialux P 0,25<br>Produit Ic 0,10 | 427 |

TABLEAU 2

## Revendications

**1 -** Benzophénone de formule générale (I)

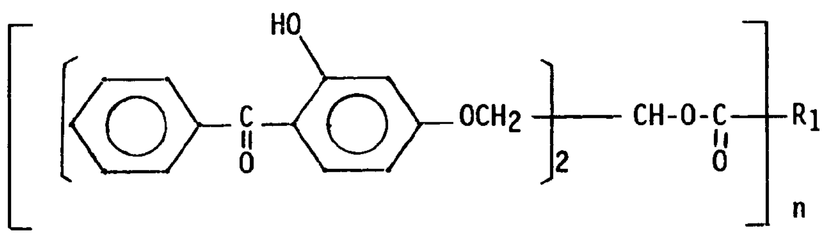

(I)

dans laquelle :

- n représente 1 ou 2
- $R_1$ lorsque n est égal à 1, de 6 à 40, avantageusement de 12 à 40 de préference de 14 à 30 atomes de carbone et représente.
  - . un radical alkyle
  - . un radical phényle,
  - . un radical phényle comportant 1 ou 2 substituants alkyles, linéaires ou ramifiés, ayant de 1 à 6 atomes de carbone, et/ou un radical hydroxyle,

et si n = 2

- $R_1$ présente de 6 à 40 avantageusement de 12 à 40 de préference de 14 à 40 atomes de carbone et représente :
  - . un radical alkyle
  - . un radical orthophénylène, métaphénylène ou paraphénylène,
  - . un radical orthophénylène, métaphénylène ou paraphénylène, comportant 1 ou 2 substituants alkyles, linéaires ou ramifiés, ayant de 1 a 6 atomes de carbone,
  - . un radical cyclohexane orthodialkylène, dont les radicaux alkylènes, identiques aux différents linéaires ou ramifiés, comportent de 1 à 12 atomes de carbone,

. un radical orthocyclohexylène, métacyclohexylène ou paracyclohexylène,

. un radical cyclohexane-dialkylène ou cyclohexylène tel que défini précédemment comportant 1 ou 2 substituants alkyles, linéaires ou ramifiés, ayant 1 à 6 atomes de carbone.

**2 -** Benzophénone selon la revendication 1, de formule générale (I) dans laquelle :

- n représente 1 ou 2,
- $R_1$ représente lorsque n = 1, un radical alkyle, linéaire ou ramifié, ayant de 5 à 30 atomes de carbone,
- $R_1$ représente, lorsque n = 2 :

. un radical alkylène, linéaire ou ramifié, ayant 1 à 24 atomes de carbone,

. un radical cyclohexane-orthodioctaméthylène comportant 2 substituants alkyles,linéaires ou ramifiés ayant 6 atomes de carbone.

**3 -** Benzophénone de formule générale (I) selon l'une des revendications 1 ou 2, choisie parmi :

- le décanoate de di(benzoyl-4 hydroxy-3 phénoxy)-1,3 propyle-2,
- le dodécanoate (laurate) de di(benzoyl-4 hydroxy-3 phénoxy)-1,3 propyle-2,
- l'hexadécanoate de di(benzoyl-4 hydroxy-3 phénoxy)-1,3 propyle-2,
- l'octadécanoate (stéarate) de di(benzoyl-4 hydroxy-3 phénoxy)-1,3 propyle-2,
- le docosanoate (béhénate) de di(benzoyl-4 hydroxy-3 phénoxy)-1,3 propyle-2,
- le (dihexyl-4,5 cyclohexane)-di(octaméthylène-carboxylate)-1,2 de bis[di(benzoyl-4 hydroxy-3 phénoxy)-1,3 propyle-2].

**4 -** Procédé de préparation de benzophénone de formule (I) selon l'une des revendications 1 à 3, caractérisé en ce que l'on fait réagir le di(benzoyl-4 hydroxy-3 phénoxy)-1,3 propan-2 ol de formule (II) :

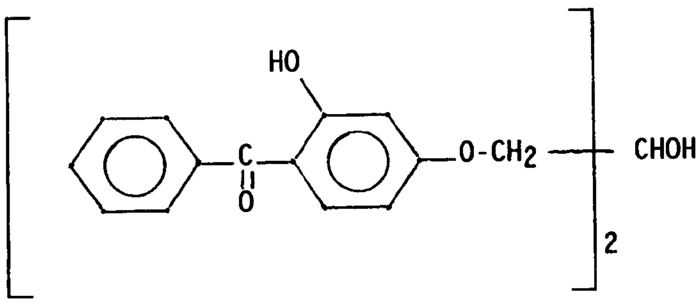

(II)

avec un acide de formule générale (III) :

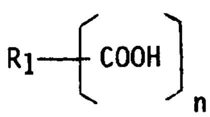

(III)

dans laquelle :

- n représente 1 ou 2
- $R_1$ a des significations indiquées précédemment pour les composés de formule générale (I),

en présence d'un catalyseur acide.

**5 -** Procédé selon la revendication 4, caractérisé en ce que la réaction est effectuée dans un solvant permettant d'éliminer par distillation azéotropique l'eau formée, comme le toluène, le benzène ou les xylènes.

**6 -** Procédé selon l'une des revendications 4 ou 5, caractérisé en ce que la réaction est conduite à une température de 50°C à 160°C et de préférence de 60°C à 130°C.

**7 -** Utilisation de benzophénone de formule (I) selon l'une des revendications 1 à 3 comme absorbeurs UV dans les polymères organiques tels que les polyoléfines, les polystyrènes, les polyalcadiènes, les polyuréthannes, les polyéthers-sulfones, les polyéthers-cétones, les polymères acryliques, les polymères halogénés, leurs copolymères et leurs mélanges, avec la condition que lorsque lesdits polymères sont ou contiennent au moins 1/3 de préference au moins 1/2, le radical $R_1$ présente avantageusement au moins 12 de préférence 14 atomes de carbone.

**8 -** Composition de polymère organique stabilisé par une quantité efficace d'au moins une benzophénone de formule (I) selon l'une des revendications 1 à 3, ayant de préférence une masse moléculaire égale ou supérieure a 600.

**9 -** Composition selon la revendication 8, de polymère organique stabilisé par une quantité efficace d'au

moins une benzophénone de formule (I) et d'au moins une benzophénone de formule générale (IV) :

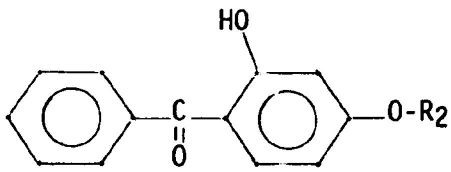

(IV)

dans laquelle $R_2$ représente un radical alkyle, linéaire ou ramifié, ayant de 1 à 30 atomes de carbone.

**10 -** Composition selon l'une des revendications 8 ou 9, caractérisée en ce qu'elle contient :

- soit de 0,005 % à 5 % en poids de benzophénone de formule (I) par rapport au poids du polymère à stabiliser et de préférence de 0,01 % à 2 % en poids par poids,
- soit de 0,005 % à 5 % en poids d'un mélange de benzophénone de formule (I) et de benzophénone de formule (IV) par rapport au poids du polymère à stabiliser et de préférence de 0,01 % à 2 % en poids par poids.

**11 -** Composition selon l'une des revendications 8 à 10 caractérisé par le fait que le dit polymère contient en masse au moins 1/3 de polyoléfine ( y compris le polystyrène) avantageusement 1/2 et par le fait que le radical $R_1$ présente avatageusement au moins 12 de préférence 14 atomes de carbone.

Office européen des brevets

# RAPPORT DE RECHERCHE EUROPEENNE

Numero de la demande

EP 92 40 1834

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | WO-A-9 104 243 (RIEDEL DE HAEN AG)<br>* page 2, ligne 4 - page 4, ligne 34 *<br>* page 12 - page 13; exemples 2,3 *<br>* page 15 - page 17; revendications 1,4,5 * | 1,2,4-8 | C07C69/24<br>C07C69/007<br>C07C67/08<br>C09K15/06 |
| X | DE-A-3 438 190 (RIEDEL DE HAEN AG)<br>* page 4, ligne 26 - page 5, ligne 22 *<br>* page 8, ligne 1 - ligne 32 *<br>* page 9, ligne 2 - ligne 12 *<br>* page 10; exemple 1 * | 1,4-8 | |

DOMAINES TECHNIQUES RECHERCHES (Int. Cl.5 )

C07C

Le présent rapport a été établi pour toutes les revendications

| Lieu de la recherche | Date d'achèvement de la recherche | Examinateur |
|---|---|---|
| LA HAYE | 27 OCTOBRE 1992 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)